# EUROPEAN PATENT APPLICATION

(11) **EP 4 806 493 A1**
(43) Date of publication of application: **16.09.2026**
(21) Application number: 24888350.6
(22) Date of filing: 28.08.2024
(51) Int. Cl.: A61M 25/09

(54) **GUIDE WIRE**

(30) Priority: 09.11.2023 JP 2023191444
(71) Applicant: ASAHI INTECC CO., LTD., Seto-shi, Aichi, 489-0071 (JP)
(72) Inventor: SATOU, Fumitaka, Seto-shi, Aichi 489-0071 (JP); INAGAKI, Motoharu, Seto-shi, Aichi 489-0071 (JP)
(74) Representative: Winter, Brandl - Partnerschaft mbB
(86) International application number: PCT/JP2024/030623
(87) International publication number: WO 2025/100048

(57) **Abstract**

A guide wire includes: a core shaft having a first region provided at a distal end portion and a second region provided on a proximal end side relative to the first region and being more easily plastically deformed than the first region; a coil body covering an outer periphery of the core shaft; and a joint part joining a distal end portion of the first region and a distal end portion of the coil body. In a longitudinal axis direction of the core shaft, a distal end of the second region is located on a proximal end side relative to a proximal end of the joint part.

## Description

### TECHNICAL FIELD

The technology disclosed herein relates to a guide wire.

### BACKGROUND ART

Guide wires used when inserting a catheter or the like into a blood vessel are known. When such a guide wire is used, a doctor may impart a curved shape to a distal end portion of the guide wire. Imparting a curved shape to the distal end portion of the guide wire by a doctor is also referred to as shaping.

### CITATION LIST

### PATENT LITERATURE

Patent Literature 1: JP 4354523 B2

### SUMMARY OF INVENTION

### TECHNICAL PROBLEM

In the guide wire described in Patent Literature 1, when a curved shape is imparted to the distal end portion of the guide wire, coil cracking may occur at a proximal end of a rigid distal end portion, and a curvature angle of a shaped part may become larger than necessary. "Coil cracking" means that a gap between wires becomes large in a part of a coil body, in other words, a part where a coil pitch is widely opened is generated. Thus, when the curvature angle of the shaped part becomes larger than necessary due to the coil cracking, the shaped part easily comes into contact with an inner wall of a combined device during delivery of the guide wire, and slidability of the guide wire in the combined device decreases. Examples of the combined device include a catheter. Such a problem is common not only to a vascular system but also to guide wires inserted into respective organs in a human body such as a lymphatic system, a biliary system, a urinary system, a respiratory system, a digestive system, a secretory gland, and a reproductive organ.

The technology disclosed herein has been made to solve at least a part of the above-described problems, and provides a guide wire capable of suppressing coil cracking at a proximal end of a joint part between a core shaft and a coil body associated with shaping.

### SOLUTION TO PROBLEM

The technology disclosed herein has been made to solve at least a part of the above-described problems, and can be realized as the following aspects.
(1) According to one aspect of the present disclosure, a guide wire is provided. This guide wire includes: a core shaft having a first region provided at a distal end portion and a second region provided on a proximal end side relative to the first region and being more easily plastically deformed than the first region; a coil body covering an outer periphery of the core shaft; and a joint part joining a distal end portion of the first region and a distal end portion of the coil body, wherein in a longitudinal axis direction of the core shaft, a distal end of the second region is located on a proximal end side relative to a proximal end of the joint part.

The technology disclosed herein can be realized in various aspects, and can be realized in forms such as a guide wire, a medical device including a guide wire, a method for manufacturing a guide wire, and a method for manufacturing a medical device including a guide wire.

### BRIEF DESCRIPTION OF DRAWINGS

Fig. 1 is an explanatory view illustrating a configuration of a guide wire according to a first embodiment.
Fig. 2 is an enlarged view of a distal end side of the guide wire.
Fig. 3 is a diagram showing a relationship between a thickness of a core film and a color.
Fig. 4 is a diagram showing an example of the core film.
Fig. 5 is a diagram illustrating a determination test of ease of plastic deformation.
Fig. 6 is a diagram showing an example of an image obtained when observing a first region with a TEM.
Fig. 7 is a diagram showing an example of an image obtained when observing a second region with a TEM.
Fig. 8 is a flowchart showing a method for manufacturing the guide wire.
Fig. 9 is an enlarged view of a distal end side of a guide wire according to a second embodiment.
Fig. 10 is a flowchart showing a method for manufacturing the guide wire according to the second embodiment.
Fig. 11 is an enlarged view of a distal end side of a guide wire according to a third embodiment.
Fig. 12 is a flowchart showing a method for manufacturing the guide wire according to the third embodiment.
Fig. 13 shows an example of a core film according to a fourth embodiment.
Fig. 14 is an enlarged view of a distal end side of a guide wire according to a fifth embodiment.

### EMBODIMENTS OF THE INVENTION

### <First Embodiment>

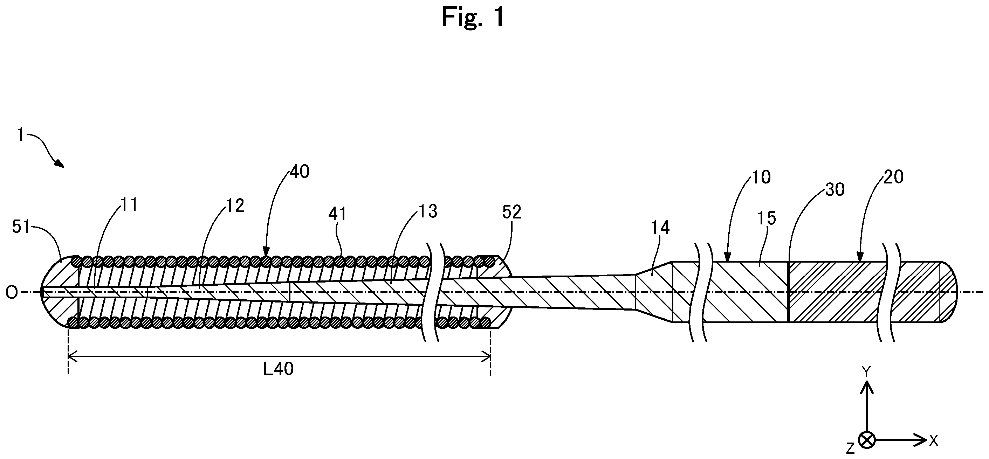

Fig. 1 is an explanatory view illustrating a configuration of a guide wire 1 according to a first embodiment. The guide wire 1 is a medical device used when inserting another medical device into a blood vessel or a digestive organ, and includes a first core shaft 10, a coil body 40, and a distal end side joint part 51. Examples of the other medical device include a catheter. The guide wire 1 includes a second core shaft 20. The guide wire 1 includes a proximal end side joint part 52. In the guide wire 1, the first core shaft 10 has first to third regions A1 to A3 described later, and the coil body 40 has fourth and fifth regions A4 and A5 described later, whereby coil cracking when the guide wire 1 is shaped can be suppressed.

"Shaping" of the guide wire 1 means an operation of imparting a curved shape to a distal end portion of the guide wire 1 mainly for improvement of vessel selectivity. Further, "coil cracking" means that a gap between wires becomes large in a part of the coil body 40, in other words, a part where a coil pitch of the coil body 40 is widely opened is generated. The first core shaft 10 corresponds to a "core shaft", and the distal end side joint part 51 corresponds to a "joint part".

In Fig. 1, for convenience of explanation, a relative ratio of sizes of respective constituent members includes a part different from an actual one. A part of each constituent member includes an exaggerated part. In Fig. 1, an axis passing through the center of the guide wire 1 is represented by an axis O. In Fig. 1, the axis O is represented by a dashed-dotted line. The axis O coincides with an axis passing through each center of the first core shaft 10, the second core shaft 20, and the coil body 40. The axis O may be different from each center axis of each constituent member described above. Fig. 1 illustrates X, Y, and Z axes orthogonal to each other. The X axis corresponds to a longitudinal direction of the guide wire 1, the Y axis corresponds to a thickness direction of the guide wire 1, and the Z axis corresponds to a width direction of the guide wire 1. The left side of Fig. 1, that is, a -X axis direction is referred to as a "distal end side" of the guide wire 1 and each constituent member, and the right side of Fig. 1, that is, a +X axis direction is referred to as a "proximal end side" of the guide wire 1 and each constituent member. Regarding the guide wire 1 and each constituent member, an end part located on the distal end side is referred to as a "distal end", and the distal end and the vicinity thereof are referred to as a "distal end portion". An end part located on the proximal end side is referred to as a "proximal end", and the proximal end and the vicinity thereof are referred to as a "proximal end portion". The distal end side is inserted into a living body, and the proximal end side is operated by a doctor. These points are common in Fig. 1 and subsequent figures.

The first core shaft 10 is arranged on the distal end side of the guide wire 1, in other words, on the distal end side relative to the second core shaft 20. The first core shaft 10 is a tapered long member having a large diameter on the proximal end side and a small diameter on the distal end side. The first core shaft 10 of the present embodiment is formed of, for example, a nickel-titanium alloy or an alloy of nickel-titanium and another metal. The first core shaft 10 has a first part 11, a second part 12, a third part 13, a fourth part 14, and a fifth part 15 in this order from the distal end side toward the proximal end side. The thickness, width, and length of each part can be arbitrarily determined. The first core shaft 10 is also referred to as a "metal wire for medical device".

The first part 11 is a part located most on the distal end side in the first core shaft 10. The first part 11 is a part where an outer diameter of the first core shaft 10 is minimum. A film made of titanium oxide is formed on a surface of a part of the first part 11 of the present embodiment. The film formed on a part of the first part 11 is also referred to as a "core film 100". A part of the first part 11 of the present embodiment is subjected to press working for improving flexibility of the distal end portion while suppressing twisting in a bending direction of the guide wire during shaping. Details will be described later.

The second part 12 is a part located between the first part 11 and the third part 13 in the first core shaft 10. The third part 13 is a part located between the second part 12 and the fourth part 14 in the first core shaft 10. The fourth part 14 is a part located between the third part 13 and the fifth part 15 in the first core shaft 10. The thickness of the second part 12 becomes thinner toward the distal end. The thickness of the third part 13 becomes thinner toward the distal end. The thickness of the fourth part 14 becomes thinner toward the distal end. A rate of change in the thickness of the second part 12, a rate of change in the thickness of the third part 13, and a rate of change in the thickness of the fourth part 14 are different from each other. The fifth part 15 is a part located most on the proximal end side in the first core shaft 10. The fifth part 15 has a substantially cylindrical shape and is a part where the thickness of the first core shaft 10 is maximum.

In the present embodiment, "substantially constant" is synonymous with "generally constant", and means that it is generally constant while allowing deviation caused by a manufacturing error or the like. Similarly, "substantially cylindrical shape/substantially frustoconical shape" is synonymous with "generally cylindrical shape/generally frustoconical shape", and means that it is generally the shape while allowing deviation caused by a manufacturing error or the like. In the present embodiment, "same" and "equal" are not limited to a case of strictly matching, but mean allowing a difference caused by a manufacturing error or the like.

The second core shaft 20 is arranged on the proximal end side of the guide wire 1, in other words, on the proximal end side relative to the first core shaft 10. The second core shaft 20 is a substantially cylindrical member having a constant outer diameter. The outer diameter of the second core shaft 20 is the same as the outer diameter of the fifth part 15 of the first core shaft 10. The second core shaft 20 of the present embodiment is formed of, for example, a stainless alloy such as SUS304 or SUS316.

A core joint part 30 is a part where the first core shaft 10 and the second core shaft 20 are joined by welding. The core joint part 30 may be formed by fixing the first core shaft 10 and the second core shaft 20 by a means different from welding, for example, a fixing tool. The core joint part 30 is formed between a proximal end surface of the fifth part 15 of the first core shaft 10 and a distal end surface of the second core shaft 20. In the illustrated example, the core joint part 30 is in a planar shape substantially perpendicular to the axis O. The core joint part 30 may be inclined with respect to the axis O. The first core shaft 10 and the second core shaft 20 are fixed by this core joint part 30.

In the first core shaft 10, the distal end sides of the first part 11, the second part 12, and the third part 13 are covered with the coil body 40. In the first core shaft 10, the proximal end side of the third part 13, the fourth part 14, and the fifth part 15 are not covered with the coil body 40 and are exposed from the coil body 40. The proximal end portion of the second core shaft 20 is used when a doctor grips the guide wire 1.

The coil body 40 is a member covering the outer periphery of the first core shaft 10. The coil body 40 is formed by a spirally wound wire 41, and has a substantially cylindrical shape. The coil body 40 may be a single thread coil formed by winding one wire in a single thread. The coil body 40 may be a multi-thread coil formed by winding a plurality of wires in multiple threads. The coil body 40 may be a single thread twisted wire coil formed by winding a twisted wire obtained by twisting a plurality of wires in a single thread. The coil body 40 may be a multi-thread twisted wire coil formed by using a plurality of twisted wires obtained by twisting a plurality of wires and winding each twisted wire in multiple threads. A wire diameter of the wire 41 of the coil body 40, an outer diameter and an inner diameter of the coil body 40, and a length of the coil body 40 can be arbitrarily determined.

The wire 41 can be formed by, for example, a stainless alloy such as SUS304 or SUS316, a nickel-titanium alloy, a piano wire, a radiopaque alloy such as a nickel-chromium alloy or a cobalt alloy, a radiopaque alloy such as gold, platinum, tungsten, or an alloy containing these elements, or a known material other than the above. Examples of the alloy containing gold, platinum, tungsten, or these elements include a platinum-nickel alloy. On a part of the proximal end side of the coil body 40, an oxide film is formed on a surface of the wire 41. A composition of the oxide film changes depending on a material of the wire 41. For example, when the wire 41 is formed of a platinum-nickel alloy, a nickel oxide film is formed on a part of the proximal end side of the coil body 40. The film formed on a part of the coil body 40 is also referred to as a "coil film 200". Details will be described later.

The distal end side joint part 51 is provided at the distal end of the guide wire 1, and integrally holds the distal end of the first part 11 of the first core shaft 10 and the distal end of the coil body 40. The proximal end side joint part 52 is directed to an intermediate part of the guide wire 1, and integrally holds a part of the third part 13 of the first core shaft 10 and the proximal end of the coil body 40. The distal end side joint part 51 is formed of an arbitrary bonding material, for example, a metal solder such as silver brazing, gold brazing, zinc, Sn-Ag alloy, or Au-Sn alloy. The proximal end side joint part 52 is formed of an arbitrary bonding material, for example, a metal solder such as silver brazing, gold brazing, zinc, Sn-Ag alloy, or Au-Sn alloy. The distal end side joint part 51 and the proximal end side joint part 52 may use the same bonding material or may use different bonding materials.

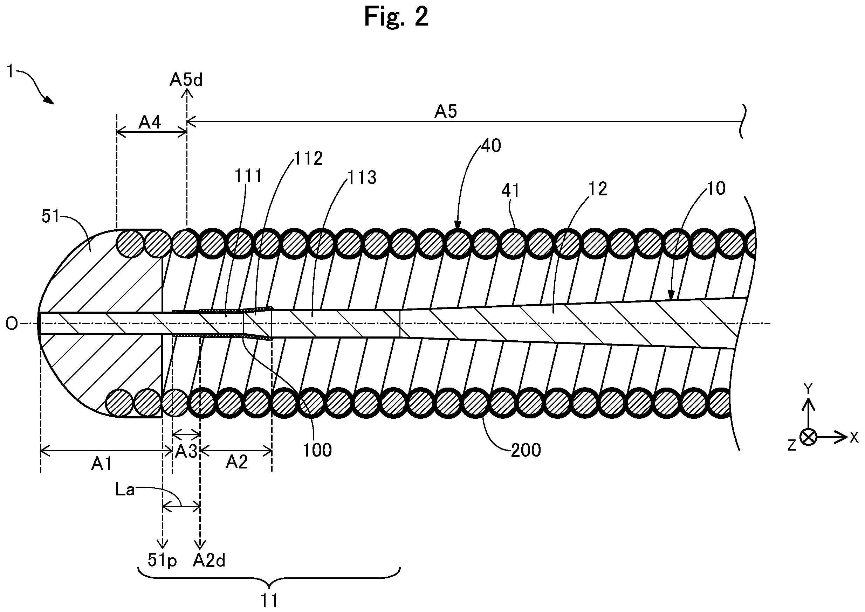

Hereinafter, the first to third regions A1 to A3 of the first core shaft 10, the fourth and fifth regions A4 and A5 of the coil body 40, and a relationship therebetween will be described with reference to Figs. 2 to 7.

Fig. 2 is an enlarged view of the distal end side of the guide wire 1. The first part 11 of the first core shaft 10 has a first press part 111, a second press part 112, and a proximal end portion 113. The first press part 111 and the second press part 112 are parts where press working is applied to a wire rod having a circular transverse section. The proximal end portion 113 is a part where press working is not applied. The second press part 112 is located between the first press part 111 and the proximal end portion 113. The second press part 112 is a part where an outer shape gradually changes from an outer shape of the first press part 111 to an outer shape of the proximal end portion 113 from the distal end side toward the proximal end side. The press working on the first press part 111 and the second press part 112 may be performed at once, or may be performed in a plurality of times. The first press part 111 and the second press part 112 are collectively referred to as a "flat part". Thus, if the flat part is formed by press working, the first part 11 of the first core shaft 10 can be made flexible.

At the distal end portion of the first core shaft 10, a first region A1, a third region A3, and a second region A2 are provided in this order from the distal end side toward the proximal end side. The first region A1 is the distal end portion of the first core shaft 10, and is provided most on the distal end side among the first to third regions A1 to A3. The first region A1 is provided at a position corresponding to the distal end portion of the first press part 111. The first region A1 is a part where the first core shaft 10 is most difficult to be plastically deformed among the first to third regions A1 to A3. As illustrated, in the first region A1, the core film 100 is not formed on the surface of the first core shaft 10.

The second region A2 is the distal end portion of the first core shaft 10, and is provided on the proximal end side relative to the first region A1 and on the proximal end side relative to the third region A3. The second region A2 is provided at a position corresponding to the proximal end portion of the first press part 111 and the second press part 112. The second region A2 is a part where the first core shaft 10 is most easily plastically deformed among the first to third regions A1 to A3. As illustrated, in the second region A2, the core film 100 is formed on the surface of the first core shaft 10. The core film 100 of the second region A2 is thicker than the core film 100 of the third region A3. A thickness (film thickness) of the core film 100 of the second region A2 can be, for example, 0.07 µm or more. The thickness of the core film 100 is also referred to as a film thickness. An operator performs observation with a TEM (transmission electron microscope) "JEM-2100F", and obtains the film thickness by measuring a thickness of a part where the film is relatively thick in an image obtained by the TEM. JEM-2100F is manufactured by JEOL Ltd.

The third region A3 is the distal end portion of the first core shaft 10, and is provided between the first region A1 and the second region A2, in other words, on the proximal end side relative to the first region A1 and on the distal end side relative to the second region A2. The third region A3 is provided at a position corresponding to an intermediate part of the first press part 111. As illustrated, the third region A3 is adjacent to the first region A1 and adjacent to the second region A2. In the third region A3, the first core shaft 10 is more easily plastically deformed than the first region A1, and the first core shaft 10 is more difficult to be plastically deformed than the second region A2. In other words, in the third region A3, the ease of plastic deformation of the first core shaft 10 is medium among the first to third regions A1 to A3. As illustrated, in the third region A3, the core film 100 is formed on the surface of the first core shaft 10. The core film 100 of the third region A3 is thinner than the core film 100 of the second region A2. The thickness of the core film 100 of the third region A3 can be, for example, 0.01 µm or more and less than 0.07 µm. A method for measuring the film thickness is as described in the second region A2.

The distal end side joint part 51 joins the distal end portion of the first region A1 of the first core shaft 10 and the distal end portion of the coil body 40. As illustrated in Fig. 2, in the longitudinal axis direction of the first core shaft 10, a distal end A2d of the second region A2 is located on the proximal end side relative to a proximal end 51p of the distal end side joint part 51. The longitudinal axis direction of the first core shaft 10 is the axis O direction. In Fig. 2, an example in which the proximal end 51p of the distal end side joint part 51 is located near the center of the first region A1 is given. The proximal end 51p of the distal end side joint part 51 may be located in an arbitrary part of the first region A1, for example, the proximal end 51p of the distal end side joint part 51 may be located at the proximal end of the first region A1. Even in this case, in the longitudinal axis direction of the first core shaft 10, the distal end A2d of the second region A2 is located on the proximal end side relative to the proximal end 51p of the distal end side joint part 51.

In the longitudinal axis direction of the first core shaft 10, the distal end A2d of the second region A2 is located within a distance of 1/5 of a total length L40 of the coil body 40 shown in Fig. 1 from the proximal end 51p of the distal end side joint part 51. In other words, a distance La between the proximal end 51p of the distal end side joint part 51 and the distal end A2d of the second region A2 satisfies a relationship of La ≤ 1/5 × L40. In the example of Fig. 2, the distal end A2d of the second region A2 and the proximal end 51p of the distal end side joint part 51 both extend perpendicularly to the axis O. For example, when the film thickness in a circumferential direction of the core film 100 is non-uniform and the distal end A2d of the second region A2 cannot be defined perpendicularly to the axis O, the distal end A2d of the second region A2 means a position of the most distal end in the second region A2. Similarly, when the distal end side joint part 51 is formed distortedly and the proximal end 51p of the distal end side joint part 51 cannot be defined perpendicularly to the axis O, the proximal end 51p of the distal end side joint part 51 means a position of the most proximal end in the distal end side joint part 51.

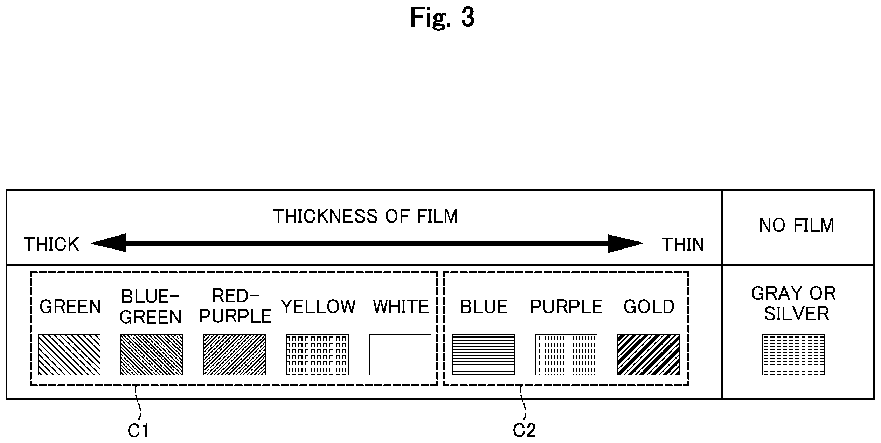

Fig. 3 is a diagram showing a relationship between the thickness of the core film 100 and the color. When heat treatment is applied to a core shaft containing titanium, such as a nickel-titanium alloy or an alloy of nickel-titanium and another metal, like the first core shaft 10 of the present embodiment, nickel and titanium are oxidized, whereby an oxide film is formed on the surface of the core shaft. The oxide film formed on the surface of the core shaft is the core film 100. The film thickness of the core film 100 becomes thicker as a heat treatment temperature is higher, and becomes thinner as the heat treatment temperature is lower. That is, the film thickness of the core film 100 is related to the heat treatment temperature. The film thickness of the core film 100 is related to a color of an external appearance of the first core shaft 10 when the first core shaft 10 is visually observed.

Specifically, as shown in Fig. 2, when the core film 100 is "green", the thickness of the film is thickest; when the core film 100 is "blue-green", the thickness of the film is second thickest; when the core film 100 is "red-purple", the thickness of the film is third thickest; when the core film 100 is "yellow", the thickness of the film is fourth thickest; when the core film 100 is "white", the thickness of the film is fifth thickest; when the core film 100 is "blue", the thickness of the film is sixth thickest; when the core film 100 is "purple", the thickness of the film is seventh thickest; and when the core film 100 is "gold", the thickness of the film is eighth thickest. When the heat treatment is not performed and the core film 100 is not formed, the color of the external appearance of the first core shaft 10 is at least one of gray and silver.

In the present embodiment, "green" includes respective colors of green system such as lime green and sky green in addition to green. "Blue-green" includes respective colors corresponding to an intermediate color between green and blue such as aquamarine and turquoise in addition to peacock green. "Red-purple" includes respective colors corresponding to an intermediate color between red and purple such as plum and magenta in addition to claret. "Yellow" includes respective colors of yellow system such as lemon yellow and topaz in addition to yellow. "White" includes respective colors of white system such as silver white and oyster white in addition to white. "Blue" includes respective colors of blue system such as cyan and navy blue in addition to blue. "Purple" includes respective colors of purple system such as violet and grape in addition to purple. "Gold" includes respective colors of gold system such as buff and beige in addition to antique gold.

The operator can visually identify the color of the core film 100 on the external appearance of the heat-treated first core shaft 10. Specifically, the operator takes an external appearance photograph of the heat-treated first core shaft 10 using a digital microscope "VHX-7000". VHX-7000 is manufactured by KEYENCE CORPORATION. The operator identifies the color of the core film 100 by visually checking the taken external appearance photograph. That is, the color of the core film 100 described above is a color under a light environment with the digital microscope VHX-7000. A confirmation place of the core film 100 is a side surface viewed from the Y axis direction. The confirmation place of the core film 100 means a photographing place of the external appearance photograph. Green, blue-green, red-purple, yellow, and white indicating that the thickness of the film is relatively thick are colors included in a "first color group C1". Blue, purple, and gold indicating that the thickness of the film is relatively thin are colors included in a "second color group C2".

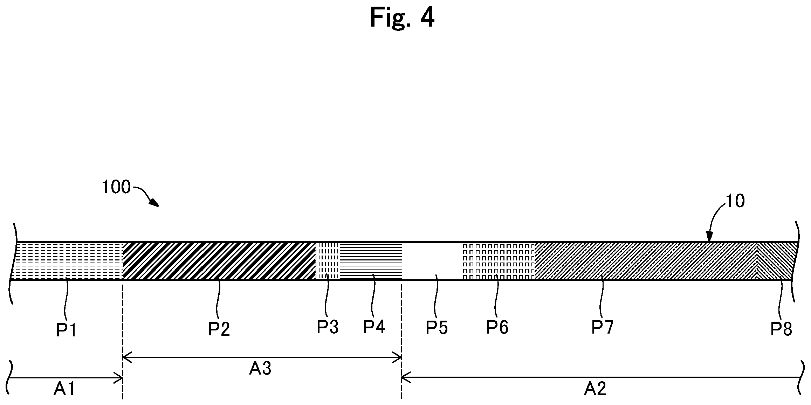

Fig. 4 is a diagram showing an example of the core film 100. Fig. 4 is a diagram representing the color of the core film 100 by hatching line types. The core film 100 of the first core shaft 10 has parts P1 to P8 from the distal end toward the proximal end. As a number attached to the end advances to 1, 2, 3, a position in the longitudinal axis direction of the first core shaft 10 moves toward the proximal end side.

As illustrated, the part P1 is silver indicating that it is not heat-treated. The part P2 has a gold film. The part P3 has a purple film. The part P4 has a blue film. The part P5 has a white film. The part P6 has a yellow film. The part P7 has a red-purple film. The part P8 has a blue-green film. In Fig. 4, for convenience of illustration, a boundary between a certain part and another part adjacent to the certain part is clearly indicated. The boundary between a certain part and another part may be a gradation in which the color of the film changes stepwise. The color of the film of each of the parts P1 to P8 and the gradation of the color at the boundary of each of the parts P1 to P8 are colors developed by the heat treatment on the core shaft. The "color of the film" includes not only the color itself but also color tone and texture.

As shown in Fig. 4, the part P1 of the first core shaft 10 does not have the core film 100 and corresponds to the first region A1. Therefore, the operator can visually identify a part where the first core shaft 10 is at least one of gray and silver as the first region A1. In the parts P5 to P8 of the first core shaft 10, the relatively thick core film 100 is formed, and the parts P5 to P8 correspond to the second region A2. Therefore, the operator can visually identify a part where the film of the first core shaft 10 exhibits a color included in the first color group C1 as the second region A2. In the parts P2, P3, and P4 of the first core shaft 10, the relatively thin core film 100 is formed, and the parts P2, P3, and P4 correspond to the third region A3. Therefore, the operator can visually identify a part where the film of the first core shaft 10 exhibits a color included in the second color group C2 as the third region A3.

The superelastic alloy constituting the first core shaft 10 has a property of being difficult to be plastically deformed (difficult to acquire a tendency to bend). Being difficult to be plastically deformed means, in other words, being difficult to acquire a tendency to bend. It is known that by applying heat treatment, the superelastic property disappears or is reduced, and it becomes easy to be plastically deformed. Therefore, in the first region A1 which is not heat-treated, the first core shaft 10 exhibits the property of the original superelastic alloy and has a property of being difficult to be plastically deformed. In the second region A2 which is strongly heat-treated and in which the relatively thick core film 100 is formed, the property of the original superelastic alloy disappears in the first core shaft 10, and the first core shaft 10 has a property of being easily plastically deformed. In the third region A3 which is located between the first region A1 and the second region A2, is weakly heat-treated, and in which the relatively thin core film 100 is formed, the property of the original superelastic alloy is reduced in the first core shaft 10, so that the first core shaft 10 has a property of being more easily plastically deformed than the first region A1 and being more difficult to be plastically deformed than the second region A2.

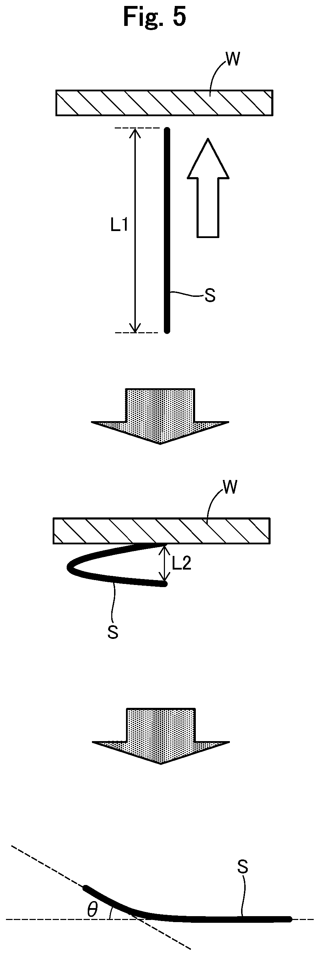

Fig. 5 is a diagram illustrating a determination test of ease of plastic deformation. The upper part of Fig. 5 shows a first procedure. The middle part of Fig. 5 shows a second procedure. The lower part of Fig. 5 shows a third procedure. First, as shown in the first procedure, a sample S of a core shaft configured entirely as any one of the first to third regions A1 to A3 is prepared. A length L1 of the sample S is 10 ± 1 mm. In the first procedure, as shown by a white arrow, one end of the sample S is pressed against a metal plate W, whereby the sample S is curved until the length becomes L2 as shown in the second procedure. The length L2 is 1 mm. Finally, as shown in the third procedure, the sample S having acquired a tendency by the second procedure is placed on a horizontal plane, and a curvature angle θ is measured.

The curvature angle θ of the first region A1 of the first core shaft 10 obtained by the determination test described in Fig. 5 is less than 1°. The curvature angle θ of the second region A2 of the first core shaft 10 similarly obtained is larger than 8°. The curvature angle θ of the third region A3 of the first core shaft 10 similarly obtained is 1° or more and 8° or less. Thus, in the first core shaft 10 of the present embodiment, it can be seen that the ease of plastic deformation has a relationship of first region A1 < third region A3 < second region A2.

In the present embodiment, the size of metal particles included in the first region A1 of the first core shaft 10 and the size of metal particles included in the second region A2 of the first core shaft 10 were respectively determined by the following procedures a1 to a7. The "size of metal particles" means an average particle size. The procedures a1 to a7 conform to JIS G0551:2020. JIS G0551:2020 corresponds to ISO 643:2012 of ISO standards and corresponds to ASTM E112-13 of ASTM standards.

(a1) An image obtained by observing an observation target region of the first core shaft 10 with a TEM is obtained. As the TEM, "JEM-2100F" was used. JEM-2100F is manufactured by JEOL Ltd. (a2) A test circle for particle counting is set for the image obtained in the procedure a1. Unlike JIS G0551:2020, here, the size of the test circle is appropriately changed, and adjustment to capture 50 or more particles in the test circle is not performed. (a3) The number N₁ of particles entirely included in the test circle set in the procedure a2 and the number N₂ of particles partially included in the test circle are counted. The number N₁ means the number of particles completely inside the test circle. The number N₂ means the number of particles intersecting the test circle. (a4) The total number of particles "N (particles) = N₁ + (N₂/2)" is obtained. (a5) The number of particles per unit area "N (particles)/test circle area (mm²)" is obtained. (a6) The reciprocal of the number of particles per unit area is taken to obtain an average area of particles. (a7) An average particle size is obtained from the average area of particles.

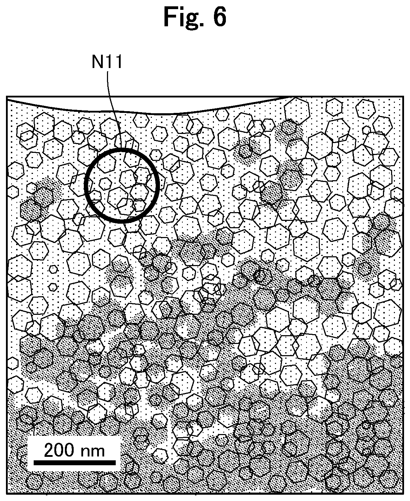

Fig. 6 is a diagram showing an example of an image obtained when observing the first region A1 of the first core shaft 10 with a TEM. Regarding a test circle N11 set at an arbitrary position, the number of particles N₁ = 15 and the number of particles N₂ = 8, and the total number of particles N = 19. Therefore, the number of particles per unit area of the test circle N11 is 605 by 9/{(0.2/2)×(0.2/2)×π}. The average area obtained from the reciprocal of the number of particles per unit area was 1.7×10⁻³ µm². Then, the average particle size obtained from the average area of particles was 5.0×10⁻² µm. The diameter of the test circle N11 is 0.2 µm.

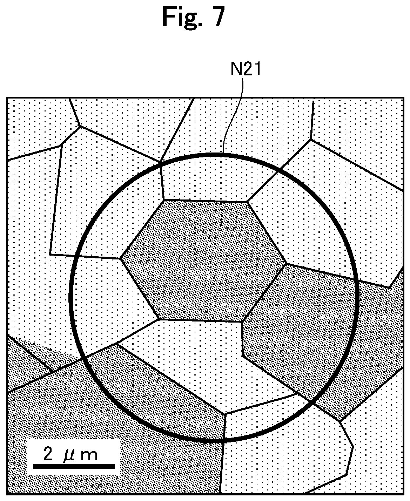

Fig. 7 is a diagram showing an example of an image obtained when observing the second region A2 of the first core shaft 10 with a TEM. Regarding a test circle N21 set at an arbitrary position, the number of particles N₁ = 5 and the number of particles N₂ = 10, and the total number of particles N = 10. The number of particles per unit area of the test circle N21 is 0.30 by 10/{(6.5/2)×(6.5/2)×π}. The average area obtained from the reciprocal of the number of particles per unit area was 3.3 µm². Then, the average particle size obtained from the average area was 2.06 µm. The diameter of the test circle N21 is 6.5 µm.

Thus, in the first core shaft 10 of the present embodiment, the size of metal particles forming the second region A2 (2.1 µm) is significantly larger than the size of metal particles forming the first region A1 (5.0×10⁻² µm). This difference in particle size contributes to the ease of plastic deformation of the first core shaft 10 in the second region A2. In the example of Fig. 5, the case where the size of metal particles forming the second region A2 of the first core shaft 10 = 2.1 µm and the size of metal particles forming the first region A1 of the first core shaft 10 = 5.0×10⁻² µm has been exemplified. This is merely an example, and the sizes of metal particles of the first region A1 and the second region A2 may be arbitrary values as long as the second region A2 > the first region A1.

Returning to Fig. 2, details of the coil body 40 will be described. In the coil body 40, a fourth region A4 and a fifth region A5 are provided in this order from the distal end side toward the proximal end side. The fourth region A4 is the distal end portion of the coil body 40, and is provided on the distal end side relative to the fifth region A5. The fourth region A4 has higher wettability of solder than the fifth region A5. As illustrated, in the fourth region A4, the coil film 200 is not formed on the surface of the coil body 40.

The fifth region A5 is provided on the proximal end side relative to the fourth region A4 from a central part to a proximal end of the coil body 40. The fifth region A5 has lower wettability of solder than the fourth region A4. This is because, as illustrated, the coil film 200 is formed on the surface of the coil body 40 in the fifth region A5.

As illustrated in Fig. 2, in the longitudinal axis direction of the first core shaft 10, the distal end A2d of the second region A2 of the first core shaft 10 is located on the proximal end side relative to a distal end A5d of the fifth region A5 of the coil body 40. In the illustrated example, the distal end A5d of the fifth region A5 is located on the proximal end side relative to the proximal end 51p of the distal end side joint part 51 and on the distal end side relative to the distal end A2d of the second region A2. In other words, the distal end A5d of the fifth region A5 is located within the range of the length La.

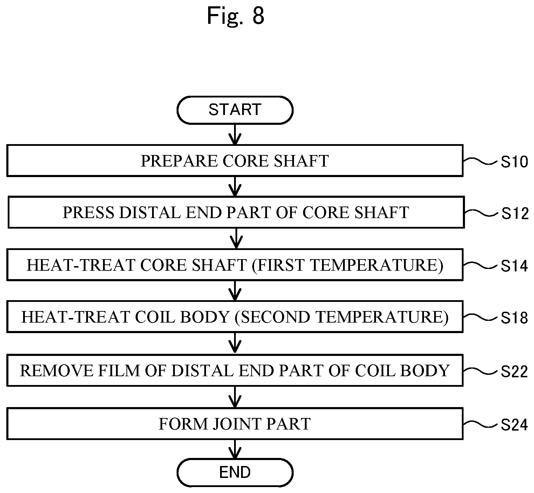

Fig. 8 is a flowchart showing a method for manufacturing the guide wire 1. In step S10, the operator prepares a core shaft made of a nickel-titanium alloy or an alloy of nickel-titanium and another metal. In step S12, the operator presses the distal end portion of the core shaft. In step S12, as described in Fig. 2, press working is performed only once to form the first press part 111, the second press part 112, and the proximal end portion 113. In step S12, press working may be performed a plurality of times.

In step S14, the operator applies heat treatment at a first temperature to an arbitrary part intended for formation of the second region A2 in the core shaft. The arbitrary part intended for formation of the second region A2 is hereinafter also referred to as a "first part". The heat treatment of step S14 is performed by "laser heat treatment" in which the core shaft is irradiated with a high-power laser and heated. The first temperature may be arbitrarily determined. The heat treatment of step S14 may be performed by another heat treatment method. Examples of the other heat treatment method include heat treatment using a heating furnace. As a result of step S14, the relatively thick core film 100, that is, the second region A2 is formed at a position corresponding to the first part in the core shaft. As a result of step S14, the relatively thin core film 100, that is, the third region A3 is formed in a part heated by heat applied to the core shaft in the heat treatment conducting on the core shaft. The part heated by heat applied to the core shaft in the heat treatment conducting on the core shaft means a part where the core shaft is heated by residual heat. A part of the core shaft where heat treatment is not performed and residual heat is not transmitted becomes the first region A1 without the core film 100. In other words, it can be said that the second region A2 and the third region A3 are a part of the heat-treated core shaft.

In step S18, the operator prepares a coil body. Thereafter, the operator applies heat treatment at a second temperature to the entire coil body. In the heat treatment of step S18, the coil body is put into a heating furnace and heated. The heat treatment of step S18 may be performed by another heat treatment method. Examples of the other heat treatment method include laser heat treatment. As a result of step S18, a film is formed on the entire coil body. In step S22, the operator removes the film formed in step S18 for an arbitrary part intended for formation of the fourth region A4 in the coil body. The arbitrary part intended for formation of the fourth region A4 is hereinafter also referred to as a "second part". By step S22, the part from which the film has been removed becomes the fourth region A4, and the part where the film remains becomes the fifth region A5. The operator can remove the film by mechanically polishing the second part which is the distal end portion of the coil body. The operator may remove the film using chemical polishing instead of mechanical polishing. In other words, it can be said that the fifth region A5 is a part of the heat-treated coil body.

In step S24, the operator forms a joint part to integrally fix the first core shaft 10 produced in steps S10 to S14 and the coil body 40 produced in steps S18 to S22. Specifically, the operator arranges the first core shaft 10 and the coil body 40 in the positional relationship described in Fig. 2, that is, an arrangement in which the distal end A2d of the second region A2 is located on the proximal end side relative to the distal end A5d of the fifth region A5. Thereafter, the operator joins the distal end portion of the first core shaft 10 and the distal end portion of the coil body 40 with an arbitrary bonding material, for example, a metal solder such as silver brazing, gold brazing, zinc, Sn-Ag alloy, or Au-Sn alloy, thereby forming the distal end side joint part 51. The coil film 200 has a property that wettability of solder is low. In step S24, since soldering is performed in an arrangement in which the distal end A2d of the second region A2 is located on the proximal end side relative to the distal end A5d of the fifth region A5, the molten solder does not wet and spread to the fifth region A5 of the coil body 40. As a result, it is possible to suppress the distal end side joint part 51 from being formed overlappingly on the second region A2 of the first core shaft 10. Since the core film 100 also has a property that wettability of solder is low similarly to the coil film 200, the presence of the core film 100 can also suppress the distal end side joint part 51 from being formed overlappingly on the second region A2 and the third region A3 of the first core shaft 10.

As described above, in the guide wire 1 of the first embodiment, as shown in Fig. 2, the first region A1 where the first core shaft 10 is difficult to be plastically deformed as compared with the second region A2 is located at the proximal end 51p of the distal end side joint part 51 joining the first core shaft 10 and the coil body 40. Therefore, even when an operation of imparting a curved shape to the distal end portion of the guide wire 1 is performed on the guide wire 1 of the present embodiment, since the first region A1 where the first core shaft 10 is difficult to be plastically deformed is located at the proximal end 51p of the distal end side joint part 51, it is possible to suppress a curved shape from being imparted to the proximal end 51p of the distal end side joint part 51. As a result, it is possible to suppress occurrence of coil cracking which has conventionally occurred along with a curved shape being imparted to a proximal end of a joint part. Coil cracking means cracking of the coil body 40. Along with suppression of coil cracking, it is possible to suppress a curvature angle of a shaped part from becoming larger than necessary, and it is possible to suppress a decrease in slidability during delivery.

According to the guide wire 1 of the first embodiment, as shown in Fig. 5, the size of metal particles forming the second region A2 of the first core shaft 10 is larger than the size of metal particles forming the first region A1. Therefore, ease of plastic deformation of the first region A1 and the second region A2 of the first core shaft 10 can be identified from the external appearance of the first core shaft 10.

Furthermore, according to the guide wire 1 of the first embodiment, the first core shaft 10 has the third region A3 between the first region A1 and the second region A2, the ease of plastic deformation of the third region A3 being larger than that of the first region A1 and smaller than that of the second region A2. Therefore, a rigidity gap of the first core shaft 10 can be alleviated, and the first core shaft 10 with gradually changed rigidity can be realized.

On the distal end side relative to the second region A2, since the first core shaft 10 is more difficult to be plastically deformed than in the second region A2, it is difficult to impart a curved shape to the guide wire 1 on the distal end side relative to the second region A2. In this regard, according to the guide wire 1 of the first embodiment, in the longitudinal axis direction of the first core shaft 10, the distal end A2d of the second region A2 is located within a distance of 1/5 of the total length L40 of the coil body 40 from the proximal end 51p of the distal end side joint part 51. Therefore, it is possible to adopt a configuration in which a curved shape can be imparted to the guide wire 1 up to a position close to the proximal end 51p of the distal end side joint part 51 while avoiding the position of the proximal end 51p of the distal end side joint part 51. As a result, it is possible to make a shaping length of the distal end portion shorter than before while suppressing occurrence of coil cracking. Then, during delivery of the guide wire, the shaped part becomes difficult to come into contact with the inner wall of the combined device, and a decrease in slidability of the guide wire in the combined device can be suppressed.

Furthermore, according to the guide wire 1 of the first embodiment, as shown in the first press part 111 and the second press part 112 of Fig. 2, the distal end portion of the first core shaft 10 has a flat shape. Therefore, twisting in the bending direction of the guide wire 1 during shaping can be suppressed. Furthermore, since the flat shape is formed by press working, the distal end portion of the guide wire 1 can be configured flexibly.

Furthermore, according to the guide wire 1 of the first embodiment, as shown in Fig. 8, the distal end portion of the first core shaft 10 is formed of a superelastic material, and the second region A2 is formed by heat-treating the first core shaft 10. Therefore, the second region A2 can be formed with pinpoint accuracy in an intended part in the entire first core shaft 10. By causing the superelastic property of the first core shaft 10 to disappear or be reduced by heat treatment, the second region A2 can be easily formed.

Furthermore, according to the guide wire 1 of the first embodiment, as shown in Fig. 2, in the longitudinal axis direction of the first core shaft 10, the distal end A2d of the second region A2 is located on the proximal end side relative to the distal end A5d of the fifth region A5 having low wettability of solder. Therefore, when the first core shaft 10 and the coil body 40 are soldered to form the distal end side joint part 51, since the solder does not wet and spread to the fifth region A5, it is possible to suppress the distal end side joint part 51 formed by the solder from being formed overlappingly on the second region A2 of the first core shaft 10. By adjusting the length of the fourth region A4 of the coil body 40 and a boundary position between the fourth and fifth regions, the length of the distal end side joint part 51 and the position of the distal end side joint part 51 can be easily adjusted. The length of the distal end side joint part 51 is a soldering length. Furthermore, if the decrease in wettability of solder in the fifth region A5 is realized by the coil film 200, corrosion of the guide wire 1 can be suppressed.

### <Second Embodiment>

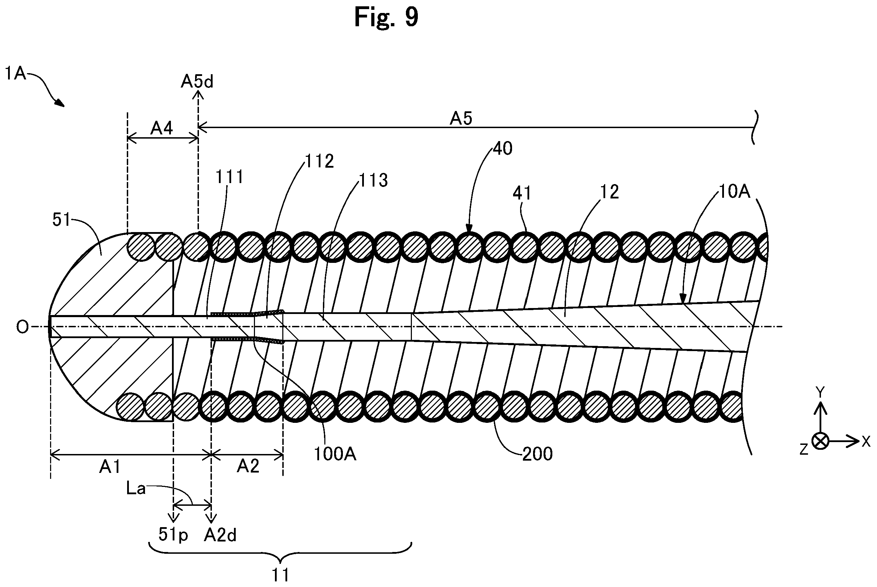

Fig. 9 is an enlarged view of a distal end side of a guide wire 1A according to a second embodiment. In the second embodiment, an example having no third region A3 will be described. The guide wire 1A of the second embodiment includes a first core shaft 10A instead of the first core shaft 10 in the configuration described in the first embodiment.

The shape of the first core shaft 10A is the same as that of the first core shaft 10 of the first embodiment. At a distal end portion of the first core shaft 10A, a first region A1 and a second region A2 are provided in this order from the distal end side toward the proximal end side. The first region A1 and the second region A2 have the same configurations as those of the first embodiment, respectively, except that they are adjacent to each other. A core film 100A of the second embodiment has only a part corresponding to the second region A2, that is, a part where the thickness of the core film 100A is relatively thick. Thus, the first core shaft 10A is not provided with the third region A3 described in the first embodiment.

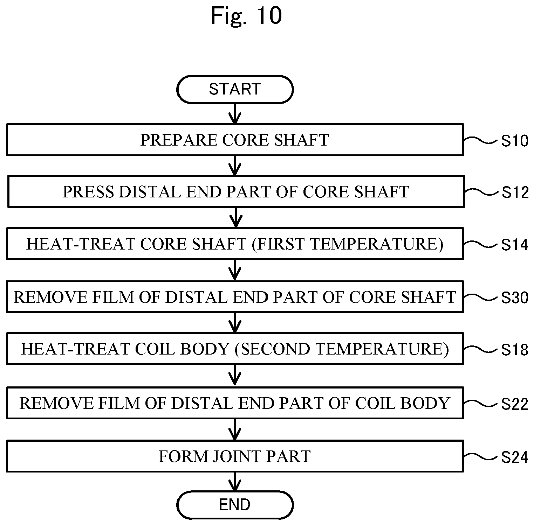

Fig. 10 is a flowchart showing a method for manufacturing the guide wire 1A according to the second embodiment. A difference from the first embodiment described in Fig. 8 is that step S30 is added between step S14 and step S18. In step S30, the operator removes the film of the distal end portion of the core shaft. Specifically, the operator removes a relatively thin core film formed by being heated by residual heat among films formed in step S14. In other words, the operator removes the third region A3 among films formed in step S14. In order to specify a removal range of the film, the operator may refer to the color of the film of the heated core shaft and remove a part where the color of the film is a color included in the second color group C2. The part where the color of the film is a color included in the second color group C2 is a part where the color of the film is blue, purple, or gold in the example of Fig. 3. Removal of the film may be performed by mechanical polishing. The operator may remove the film using chemical polishing instead of mechanical polishing.

Thus, the configuration of the guide wire 1A can be variously changed, and the first core shaft 10A may have only the first region A1 and the second region A2 and does not have to have the third region A3. Also in such a guide wire 1A of the second embodiment, effects similar to those of the first embodiment described above can be obtained.

### <Third Embodiment>

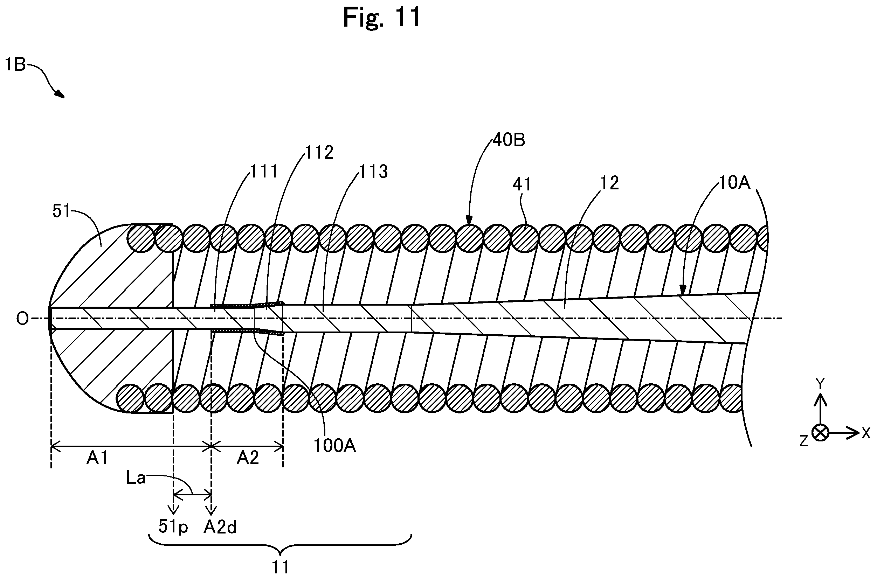

Fig. 11 is an enlarged view of a distal end side of a guide wire 1B according to a third embodiment. In the third embodiment, an example having no coil film 200 will be described. The guide wire 1B of the third embodiment includes a coil body 40B instead of the coil body 40 in the configuration described in the second embodiment of Fig. 9.

The coil film 200 described in the first embodiment is not formed on the coil body 40B. That is, the coil body 40 does not have the fourth region A4 and the fifth region A5 described in the first embodiment.

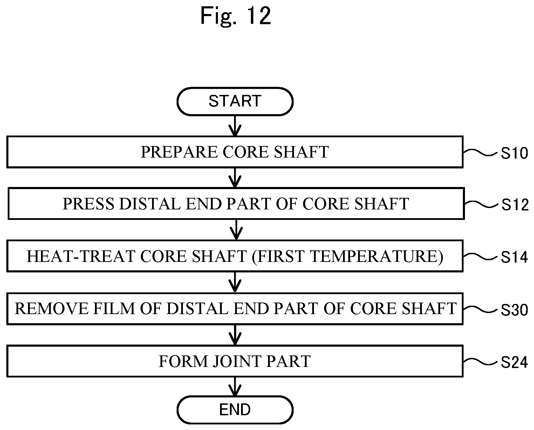

Fig. 12 is a flowchart showing a method for manufacturing the guide wire 1B according to the third embodiment. A difference from the second embodiment described in Fig. 10 is that steps S18 to S22 of processing the coil body are not executed. Also by the manufacturing method of Fig. 12, the core film 100A covering the surface of the first core shaft 10A suppresses the solder from wetting and spreading to the second region A2 of the first core shaft 10A in step S24.

Thus, the configuration of the guide wire 1B can be variously changed, and the coil body 40B may not have the fourth region A4 and the fifth region A5, and wettability of solder may be constant. Also in such a guide wire 1B of the third embodiment, effects similar to those of the first and second embodiments described above can be obtained.

### <Fourth Embodiment>

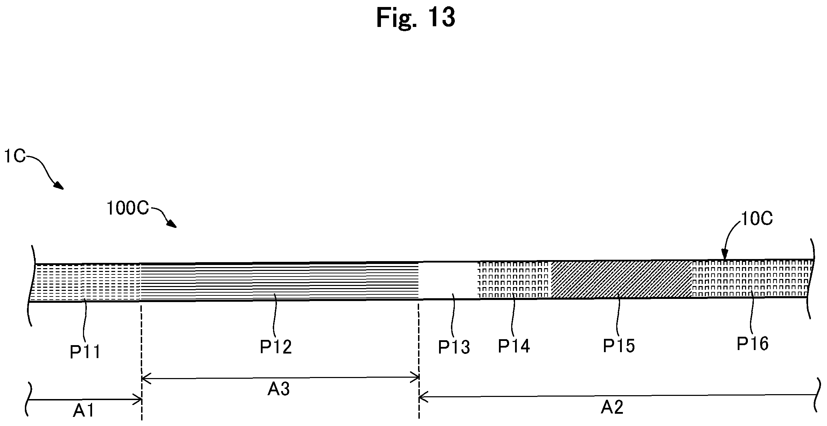

Fig. 13 shows an example of a core film 100C according to a fourth embodiment. In the fourth embodiment, an example in which the color of the core film 100C is different from that of the first embodiment will be described. A guide wire 1C of the fourth embodiment includes a first core shaft 10C instead of the first core shaft 10 in the configuration described in the first embodiment. On a surface of the first core shaft 10C, the core film 100C shown in Fig. 13 is formed. Fig. 13 is a diagram representing the color of the core film 100C by hatching line types, similarly to Fig. 4.

As shown in Fig. 13, the second region A2 of the first core shaft 10C has a white part P13, a yellow part P14, a red-purple part P15, and a yellow part P16 from the distal end side toward the proximal end side. Thus, a pattern of colors appearing in the second region A2 may be different from that of the first embodiment. The same color may appear repeatedly as in the parts P14 and P16. The third region A3 of the first core shaft 10C is entirely a blue part P12. Thus, the color appearing in the third region A3 may be a single color.

Thus, the configuration of the core film 100C can be variously changed, and the first region A1, the second region A2, and the third region A3 may have an arbitrary number of colors and an arbitrary color pattern as long as they have the colors described in Fig. 3, respectively. Specifically, it may be arbitrarily changed as long as a film of at least one color included in the first color group C1 is formed in the second region A2 and a film of at least one color included in the second color group C2 is formed in the third region A3. A boundary between a certain color and another color does not need to be clearly defined, and may be a gradation. Also in such a guide wire 1C of the fourth embodiment, effects similar to those of the first embodiment described above can be obtained.

### <Fifth Embodiment>

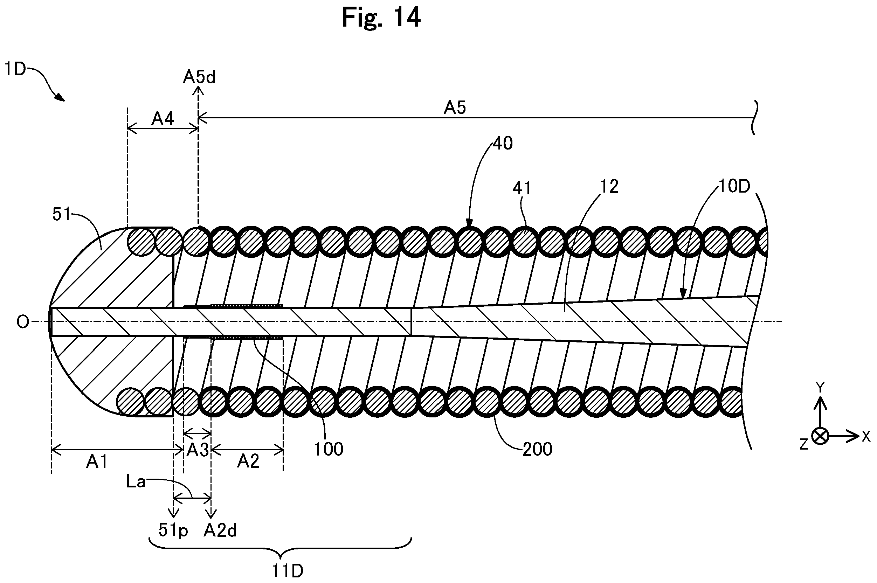

Fig. 14 is an enlarged view of a distal end side of a guide wire 1D according to a fifth embodiment. In the fifth embodiment, an example in which a distal end portion of a first core shaft 10D is not flat will be described. The guide wire 1D of the fifth embodiment includes the first core shaft 10D instead of the first core shaft 10 in the configuration described in the first embodiment. The first core shaft 10D has a first part 11D instead of the first part 11.

The first part 11D is not pressed at a distal end portion and has a cylindrical shape. In other words, the first part 11D does not have the first press part 111 and the second press part 112 described in the first embodiment. The manufacture of the guide wire 1D may be performed by omitting the press working step S12 of the distal end portion in the manufacturing method described in Fig. 8.

Thus, the configuration of the guide wire 1D can be variously changed, and the shape of each member can be arbitrarily changed. In addition to the above, a part of the second part 12, the third part 13, and the fourth part 14 of the first core shaft 10 may be omitted. An inner coil body may be provided between the first core shaft 10 and the coil body 40, and the distal end side joint part 51 may be configured to fix a distal end portion of the inner coil body in addition to the first core shaft 10 and the coil body 40.

### <Modifications of Present Embodiment>

The present disclosure is not limited to the above embodiments, and can be implemented in various aspects without departing from the gist thereof, and for example, the following modifications are possible.

### [Modification 1]

In the first to fifth embodiments described above, the configurations of the guide wires 1, 1A to 1D have been exemplified. The configuration of the guide wire 1 can be variously changed. For example, the guide wire 1 may further include a coating layer formed of at least one of a hydrophilic resin and a hydrophobic resin on surfaces of the first core shaft 10, the second core shaft 20, and the coil body 40. For example, a length in a longitudinal direction of the coil body 40, in other words, a range in which the coil body 40 covers the first core shaft 10 may be arbitrarily changed. For example, the guide wire 1 may further include an intermediate joint part for joining the first core shaft 10 and the coil body 40 between the distal end side joint part 51 and the proximal end side joint part 52. For example, the shape of the second core shaft 20 can be variously changed. The guide wire 1 may not have the second core shaft 20.

In the above embodiment, the first press part 111 and the second press part 112 as the flat parts are formed by press working. The flat part may be realized by using a wire rod having a rectangular transverse section for the first part 11 regardless of press working. The proximal end portion 113 may also be subjected to press working. In this case, the proximal end portion 113 is also included in the flat part.

In the above embodiment, the method for manufacturing the guide wires 1, 1A to 1D has been exemplified. The method for manufacturing the guide wire 1 can be variously changed. For example, the heat treatment of the core shaft and the heat treatment of the coil body may be realized by various known methods in addition to laser heat treatment and heating using a heating furnace. The first temperature which is the heat treatment temperature of the core shaft and the second temperature which is the heat treatment temperature of the coil body may be the same, or the first temperature may be lower than the second temperature. For example, the method for manufacturing the guide wire 1 may include other steps not described above, or the execution order of the steps may be changed. Examples of the other steps include a first determination step of determining whether or not the second region A2 is formed by inspecting the color of the core film 100, and a second determination step of determining whether or not the fifth region A5 is formed by inspecting the color of the coil film 200.

### [Modification 2]

In the first to fifth embodiments described above, the configurations of the first core shafts 10, 10A to 10D have been exemplified. The configuration of the first core shaft 10 can be variously changed. For example, the shape of the first core shaft 10 described in Fig. 1 is merely an example, and various changes are possible.

For example, in the longitudinal axis direction of the first core shaft 10, the distal end A2d of the second region A2 may not be located within a distance of 1/5 of the total length L40 of the coil body 40 from the proximal end 51p of the distal end side joint part 51. In other words, the distal end A2d of the second region A2 may be located on the proximal end side relative to 1/5 of the total length L40 of the coil body 40 from the proximal end 51p of the distal end side joint part 51.

For example, the thickness of the core film 100 of the first core shaft 10 described above is merely an example, and various changes are possible. For example, the thickness of the core film 100 of the second region A2 of the first core shaft 10 may be, for example, smaller than 0.07 µm. Similarly, the thickness of the core film 100 of the third region A3 may be, for example, smaller than 0.01 µm, or may be larger than 0.07 µm.

For example, the curvature angle of the first core shaft 10 described above is merely an example, and various changes are possible. For example, the curvature angle of the first region A1 of the first core shaft 10 may be 1° or more. The curvature angle of the second region A2 of the first core shaft 10 may be 8° or less. The curvature angle of the third region A3 of the first core shaft 10 may be outside the range of 1° or more and 8° or less.

### [Modification 3]

The configurations of the guide wires 1, 1A to 1D and the first core shafts 10, 10A to 10D of the first to fifth embodiments and the configurations of the guide wires 1, 1A to 1D and the first core shafts 10, 10A to 10D of Modifications 1 and 2 may be combined as appropriate. For example, in the configuration of any one of the second embodiment described for the example without the third region A3 and the third embodiment described for the example without the fourth region A4 and the fifth region A5, the film color of the fourth embodiment may be applied, or the core shaft shape of the fifth embodiment may be applied. For example, the third embodiment has been described as a modification of the second embodiment. The third embodiment may be realized as a modification of any one of the first, fourth, and fifth embodiments.

The present aspect has been described above based on the embodiments and modifications. The embodiments of the aspect described above are for facilitating understanding of the present aspect, and do not limit the present aspect. The present aspect can be changed and improved without departing from the gist and the scope of claims, and equivalents thereof are included in the present aspect. If the technical feature is not described as essential herein, it can be deleted as appropriate.

## Claims

1. A guide wire (1, 1A to 1D) comprising:
a core shaft (10, 10A to 10D) having a first region (A1) provided at a distal end portion and a second region (A2) provided on a proximal end side relative to the first region (A1) and being more easily plastically deformed than the first region (A1);
a coil body (40, 40B) covering an outer periphery of the core shaft (10, 10A to 10D); and
a joint part (51) joining a distal end portion of the first region (A1) and a distal end portion of the coil body (40, 40B),
wherein in a longitudinal axis direction of the core shaft (10, 10A to 10D), a distal end of the second region (A2) is located on a proximal end side relative to a proximal end of the joint part (51).

2. The guide wire (1, 1A to 1D) according to claim 1, wherein
a size of metal particles forming the second region (A2) is larger than a size of metal particles forming the first region (A1).

3. The guide wire (1, 1C, 1D) according to claim 1 or 2, wherein
the core shaft (10, 10C, 10D) further has a third region (A3) provided between the first region (A1) and the second region (A2), and
the third region (A3) is more easily plastically deformed than the first region (A1) and is more difficult to be plastically deformed than the second region (A2).

4. The guide wire (1, 1A to 1D) according to any one of claims 1 to 3, wherein
in the longitudinal axis direction of the core shaft (10, 10A to 10D), the distal end of the second region (A2) is located within a distance of 1/5 of a total length of the coil body (40, 40B) from the proximal end of the joint part (51).

5. The guide wire (1, 1A to 1C) according to any one of claims 1 to 4, wherein the distal end portion of the core shaft (10, 10A to 10C) has a flat shape.

6. The guide wire (1, 1A to 1D) according to any one of claims 1 to 5, wherein
the distal end portion of the core shaft (10, 10A to 10D) is formed of a superelastic material, and
the second region (A2) is a heat-treated part of the core shaft (10, 10A to 10D).

7. The guide wire (1, 1A, 1C, 1D) according to any one of claims 1 to 6, wherein
the coil body (40) has a fourth region (A4) provided at the distal end portion and a fifth region (A5) provided on a proximal end side relative to the fourth region (A4) and having lower wettability of solder than the fourth region (A4), and
in the longitudinal axis direction of the core shaft (10, 10A, 10C, 10D), the distal end of the second region (A2) is located on a proximal end side relative to a distal end of the fifth region (A5).
